Europäisches Patentamt

European Patent Office

Office européen des brevets

(19)

(11) Veröffentlichungsnummer : **0 344 537 B1**

(12)

# EUROPÄISCHE PATENTSCHRIFT

(45) Veröffentlichungstag der Patentschrift :
**11.03.92 Patentblatt 92/11**

(51) Int. Cl.⁵ : **C08J 9/14,** C08J 9/00,
// C08L75/04

(21) Anmeldenummer : **89109004.5**

(22) Anmeldetag : **19.05.89**

(54) **Verwendung von 1,1,1,4,4,4-Hexafluorbutan als Treib- und Dämmgas für die Herstellung von Kunststoff-Schaumstoffen.**

(30) Priorität : **01.06.88 DE 3818692**

(43) Veröffentlichungstag der Anmeldung :
**06.12.89 Patentblatt 89/49**

(45) Bekanntmachung des Hinweises auf die Patenterteilung :
**11.03.92 Patentblatt 92/11**

(84) Benannte Vertragsstaaten :
**BE CH DE ES FR GB IT LI NL SE**

(56) Entgegenhaltungen :
**US-A- 3 706 683**
**JOURNAL OF THE CHEMICAL SOCIETY, Part III, 1952, Seiten 2449-4088, London, GB; "The addition of free radicals to unsaturated systems. Part I."**

(73) Patentinhaber : **BAYER AG**
**W-5090 Leverkusen 1 Bayerwerk (DE)**

(72) Erfinder : **Lamberts, Wilhelm, Dr.**
**Roggendorfstrasse 61**
**W-5000 Köln 80 (DE)**
Erfinder : **Sommerfeld, Klaus-Dieter, Dr.**
**Büscherhöfchen 11**
**W-5063 Overath (DE)**
Erfinder : **Bielefeldt, Dr.**
**Beuthener Strasse 13**
**W-4030 Ratingen 6 (DE)**
Erfinder : **Marhold, Albrecht, Dr.**
**Carl-Duisberg-Strasse 329**
**W-5090 Leverkusen 1 (DE)**
Erfinder : **Negele, Michael, Dr.**
**Wolfskaul 6**
**W-5000 Köln 80 (DE)**

EP 0 344 537 B1

## Beschreibung

Die vorliegende Erfindung betrifft die Verwendung des Dämmgases und Treibmittels 1,1,1,4,4,4-Hexafluorbutan (R 356) für die Herstellung von Kunststoff-Schaumstoffen, insbesondere Schaumstoffen auf Isocyanatbasis, vorzugsweise Polyurethanschaumstoffen.

Es ist bekannt, daß Schaumstoffe unter Verwendung von Treibmitteln hergestellt werden. In geschlossenzelligen Schaumstoffen sind diese darüber hinaus als wärmedämmendes Zellgas wirksam. Zu den heute am weitesten verbreiteten Dämm- und Treibgasen für Schrumstoffe aus Polyurethan, Polystyrol, Polyvinylchlorid, Phenol-Formaldehyd u.a. gehören die Fluorchlorkohlenwasserstoffe Trichlorfluormethan (R 11), Dichlordifluormethan (R 12), Trichlorfluorethan (R 113) u.a..

Diese Produkte haben indessen den nachteil, daß sie infolge ihrer hohen Stabilität in die Stratosphäre gelangen, wo sie aufgrund ihres Gehaltes an Chlor zum Abbau des dort vorhandenen Ozons beitragen sollen. Aus diesem Grunde wurde die Weltproduktion an Fluorchlorkohlenwasserstoffen begrenzt.

Der Erfindung liegt die Aufgabe zugrunde, Dämmgase und Treibmittel für die Herstellung von Kunststoff-Schaumstoffen aufzufinden, die die Ozonschicht nicht schädigen können.

Diese Aufgabe wird mit dem erfindungsgemäßen Dämm- und Treibgas 1,1,1,4,4,4-Hexafluorbutan gelöst.

Gegenstand der Erfindung ist die Verwendung von 1,1,1,4,4,4-Hexafluorbutan als Treib- und Dämmgas bei der Herstellung von Kunststoff-Schaumstoffen. Gemäß einer bevorzugten Ausführungsform der Erfindung wird 1,1,1,4,4,4-Hexafluorbutan in einer Menge 2-30 Gew.-%, vorzugsweise in einer Menge von 2-15 Gew.-%, insbesondere in einer Menge von 2-8 Gew.-%, bezogen auf den Kunststoff-Schaumstoff, eingesetzt.

Bevorzugt ist erfindungsgemäß die Herstellung von Schaumstoffen auf Isocyanatbasis, insbesondere die Herstollung von Polyurothanschaumstoffen und/oder die Herstellung von Polyisocyanuratschaumstoffen.

Die Herstellung von Kunststoff-Schaumstoffen unter Verwendung von Treibgasen ist allgemein bekannt.

Auch die Herstellung von Schaumstoffen auf Isocyanatbasis ist an sich bekannt und z.B. in den deutschen Offenlegungsschriften 1 694 142, 1 694 215 und 1 720 768 sowie im Kunststoff-Handbuch Band VII, Polyurethane, herausgegeben von Vieweg und Höchtlen, Carl Hanser Verlag München 1966 sowie in der neuauflage dieses Buches, herausgegeben von G. Oertel, Carl Hanser Verlag München, Wien 1983, beschrieben.

Es handelt sich dabei vorwiegend um Urethan- und/oder Isocyanurat- und/oder Allophanat- und/oder Uretdionund/oder Harnstoff- und/oder Carbodiimidgruppen aufweisende Schaumstoffe. Die erfindungsgemäße Verwendung erfolgt vorzugsweise bei der Herstellung von Polyurethanund Polyisocyanuratschaumstoffen.

Für die Herstellung der Schaumstoffe auf Isocyanatbasis werden eingesetzt:

1. Als Ausgangskomponenten aliphatische, cycloaliphatische, araliphatische, aromatische und heterocyclische Polyisocyanate, wie sie z.B. von W. Siefken in Justus Liebigs Annalen der Chemie, 562, Seiten 75 bis 136, beschrieben werden, beispielsweise solche der Formel

$$Q(NCO)_n$$

in der

$$n = 2\text{-}4, \text{ vorzugsweise } 2\text{-}3,$$

und

Q einen aliphatischen Kohlenwasserstoffrest mit 2-18, vorzugsweise 6-10 C-Atomen, einen cycloaliphatischen Kohlenwasserstoffrest mit 4-15, vorzugsweise 5-10 C-Atomen, einen aromatischen Kohlenwasserstoffrest mit 6-15, vorzugsweise 6-13 C-Atomen oder einen araliphatischen Kohlenwasserstoffrest mit 8-15, vorzugsweise 8-13 C-Atomen bedeuten, z.B. solche Polyisocyanate, wie sie in der DE-OS 28 32 253, Seiten 10-11, beschrieben werden. Besonders bevorzugt werden in der Regel die technisch leicht zugänglichen Polyisocyanate, z.B. das 2,4- und 2,6-Toluylendiisocyanat, sowie beliebige Gemische dieser Isomeren ("TDI"); Polyphenylpolymethylenpolyisocyanate, wie sie durch Anilin-Formaldehyd-Kondensation und anschließende Phosgenierung hergestellt werden ("rohes MDI") und Carbodiimidgruppen, Urethangruppen, Allophanatgruppen, Isocyanuratgruppen, Harnstoffgruppen oder Biuretgruppen aufweisenden Polyisocyanate ("modifizierte Polyisocyanate"), insbesondere solche modifizierten Polyisocyanate, die sich vom 2,4- und/oder 2,6-Toluylendiisocyanat bzw. vom 4,4'- und/oder 2,4'-Diphenylmethandiisocyanat ableiten.

2. Ausgangskomponenten sind ferner Verbindungen mit mindestens zwei gegenüber Isocyanaten reaktionsfähigen Wasserstoffatomen von einem Molekulargewicht in der Regel von 400-10.000. Hierunter versteht man neben Aminogruppen, Thiogruppen oder Carboxylgruppen aufweisende Verbindungen vorzugsweise Hydroxylgruppen aufweisende Verbindungen, insbesondere 2 bis 8 Hydroxylgruppen aufweisende Verbindungen, speziell solche vom Molekulargewicht 1000 bis 6000, vorzugsweise 2000 bis 6000, z.B. mindestens 2, in der Regel 2 bis 8, vorzugsweise aber 2 bis 6, Hydroxylgruppen aufweisende Polyether und Polyester sowie Polycarbonate und Polyesteramide, wie sie für die Herstellung von homogenen und von zellförmigen Polyurethanen an sich bekannt sind und wie sie z.B. in der DE-OS 28 32 253, Seiten 11-18, beschrieben werden.

3. Gegebenenfalls sind weitere Ausgangskomponenten Verbindungen mit mindestens zwei gegenüber Isocyanaten reaktionsfähigen Wasserstoffatomen und einem Molekulargewicht von 32 bis 399. Auch in diesem Fall versteht man hierunter Hydroxylgruppen und/oder Aminogruppen und/oder Thiolgruppen und-/oder Carboxylgruppen aufweisende Verbindungen, vorzugsweise Hydroxylgruppen und/oder Aminogruppen aufweisende Verbindungen, die als Kettenverlängerungsmittel oder Vernetzungsmittel dienen. Diese Verbindungen weisen in der Regel 2 bis 8, vorzugsweise 2 bis 4, gogenüber Isocyanaten reaktionsfähige Wasserstoffatome auf. Beispiele hierfür werden in der DE-OS 28 32 253, Seiten 19-20, beschrieben.

4. 1,1,1,4,4,4-Hexafluorbutan als Treib- und Dämmgas.

5. Gegebenenfalls werden Hilfs- und Zusatzmittel mitverwendet wie

a) Wasser und/oder andere leicht flüchtige organische Substanzen als Treibmittel,

b) zusätzliche Katalysatoren der an sich bekannten Art in Mengen von bis zu 10 Gew.-%, bezogen auf die Mengen an Komponente 2,

c) oberflächenaktive Zusatzstoffe, wie Emulgatoren und Schaumstabilisatoren,

d) Reaktionsverzögerer, z.B. sauer reagierende Stoffe wie Salzsäure oder organische Säurehalogenide, ferner Zellregler der an sich bekannten Art wie Paraffine oder Fettalkohole oder Dimethylpolysiloxane sowie Pigmente oder Farbstoffe und weitere Flammschutzmittel der an sich bekannten Art, z.B. Trikresylphosphat, ferner Stabilisatoren gegen Alterungs- und Witterungseinflüsse. Weichmacher und fungistatisch und bakteriostatisch wirkende Substanzen sowie Füllstoffe wie Bariumsulfat, Kieselgur, Ruß-oder Schlämmkreide.

Diese gegebenenfalls mitzuverwendenden Hilfs- und Zusatzstoffe werden beispielsweise in der DE-OS 27 32 292, Seiten 21-24, beschrieben.

Weitere Beispiele von gegebenenfalls erfindungsgemäß mitzuverwendenden oberflächenaktiven Zusatzstoffen und Schaumstabilisatoren sowie Zellreglern, Reaktionsverzögerern, Stabilisatoren, flammhemmenden Substanzen, Weichmachern, Farbstoffen und Füllstoffen sowie fungistatisch und bakteriostatisch wirksamen Substanzen sowie Einzelheiten über Verwendungs- und Wirkungsweise dieser Zusatzmittel sind im Kunststoff-Handbuch, Band VII, herausgegeben von Vieweg und Höchtlen, Carl-Hanser-Verlag, München 1966, z.B. auf den Seiten 103-113 beschrieben.

Die Schaumstoffe auf Isocyanatbasis werden in an sich bekannter Weise hergestellt.

Durchführung des Verfahrens zur Herstellung von Polyurethankunststoffen: Die Reaktionskomponenten werden nach dem an sich bekannten Einstufenverfahren, dem Prepolymerverfahren oder dem Semiprepolymerverfahren zur Umsetzung gebracht, wobei man sich oft maschineller Einrichtungen bedient, z.B. solcher, die in der US-PS 27 64 565 beschrieben werden. Einzelheiten über Verarbeitungseinrichtungen, die auch erfindungsgemäß in Frage kommen, werden im Kunststoff-Handbuch, Band VII, herausgegebon von Vieweg und Höchtlen, Carl-Hanser-Verlag, München 1966, z.B. auf den Seiten 121 bis 205, beschrieben.

Erfindungsgemäß lassen sich auch kalthärtende Schaumstoffe herstellen (vgl. GB-PS 11 62 517, DE-OS 21 53 086).

Selbstverständlich können aber auch Schaumstoffe durch Blockverschäumung oder nach dem an sich bekannten Doppeltransportbandverfahren hergestellt werden.

Die nach der Erfindung erhältlichen Produkte finden z.B. als Dämmplatten für die Dachisolierung Anwendung.

I. Herstellung von 1,1,1,4,4,4-Hexafluorbutan

Beispiel A

In einem Edelstahlautoklaven wurden 40 g 1,1,1,4,4,4-Hexafluor-2-chlor-buten-2 1) in 300 ml Ethanol in Gegenwart von 12 g Kaliumhydroxid und 25 g Raney-nickel 3 Stunden lang bei 20°C und 1 Stunde bei 100°C mit Wasserstoff hydriert. Der Druck lag dabei im Bereich von 30 bis 40 bar. Danach wurden aus dem Reaktionsgemisch die festen Bestandteile durch Filtration abgetrennt und die verbleibende Flüssigkeit durch Destillation aufgearbeitet. Es wurden 16 g 1,1,1,4,4,4-Hexafluorbutan mit einem siedepunkt von 25 bis 30°C bei 1013 mbar erhalten. Das Massenspektrum zeigte für $m^+/e$ den Wert 166.

1)(siehe "Preparation, Properties and Technology of Fluorine and Organic Fluoro Compounds", Editors CH. Slessner and S.R. Schram, McGraw-Hill Bock Company, Inc., New York, Toranto, London 1951, Seite 817)

### Beispiel B

199 g (1 Mol) 1,1,1,4,4,4-Hexafluor-2-chlor-buten-2 wurden in 800 ml Diglyme und in Gegenwart von 45 g Natriumhydroxid und 30 g Raney-Nickel im Temperaturbereich von 20 bis 40°C und bei einem Wasserstoffdruck von 20 bis 40 bar hydriert. Nach dem Abfiltrieren der festen Bestandteile wurde das Lösungsmittel mit Wasser extrahiert und die abgetrennte organische Phase durch fraktionierte Destillation gereinigt. Die Ausbeute an 1,1,1,4,4,4-Hexafluorbutan betrug 125 g (75 % d.Th.), der Siedepunkt 24 bis 27°C bei 1013 mbar. Das $^{19}$F-NMR-Spektrum zeigte einen peak bei -10,7 ppm (Standard: $CF_3CO_2H$).

### Beispiel C

10 g (36 mMol) 1,1,1,4,4,4-Hexafluor-2-brom-3-chlor-buten-2 (siehe US Patent 3 567 788) wurden in 50 ml Tetrahydrofuran in Gegenwart von 3,0 g Natriumhydroxid und 5 g Raney-Nickel im Temperaturbereich von 20 bis 40°C und bei einem Wasserstoffdruck von 20 bis 40 bar hydriert. Die Aufarbeitung erfolgte wie in Beispiel B beschrieben. Die Ausbeute betrug 3,5 g 1,1,1,4,4,4-Hexafluorbutan (= 59 %. d.Th.).

### Beispiel D

40 g (0,2 Mol) 1,1,1,4,4,4-Hexafluor-2-chlor-buten-2 wurden mit 12 g Kaliumhydroxid in 300 ml Ethanol im Druckbereich von 20 bis 40 bar und bei einer Temperatur von 20 bis 100°C in Gegenwart von 25 g Raney-Nickel hydriert. Nach dem Abfiltrieren der festen Bestandteile wurde das Lösungsmittel mit Wasser extrahiert und die abgetrennte organische Phase durch Destillation gereinigt. Es wurden 15,5 g (= 47 %. d.Th.) 1,1,1,4,4,4-Hexafluorbutan erhalten. Der Siedepunkt betrug 24 bis 27°C bei 1013 bar.

### Beispiel E

23,5 g (0,1 Mol) 1,1,1,4,4,4-Hexafluor-2,3-dichlor-buten-2 (siehe US Patent 3 567 788) wurden mit 50 ml Tetrahydrofuran, 8,5 g Natriumhydroxid und 3 g eines Katalysators versetzt, der 5 Gew.-% Palladium auf Kohle enthielt. Dieses Gemisch wurde bei Temperaturen zwischen 20 und 40°C mit Wasserstoff bei Drucken im Bereich 20 bis 40 bar hydriert. Die Aufarbeitung des Reaktionsgemisches erfolgte wie in Beispiel B beschrieben. Die Ausbeute betrug 8,0 g(= 75 %. d.Th.) 1,1,1,4,4,4-Hexafluorbutan.

### II. Herstellung von Polyurethanschaumstoffen

### Beispiel 1

100 g eines Polyethers der Hydroxylzahl 380, welcher durch Anlagerung von Propylenoxid an eine Lösung aus Saccharose, Propylenglykol und Wasser erhalten wurde,

2 g eines Siloxanpolyethercopolymers als Schaumstabilisator,

3,8 g Wasser und

3 g Dimethylcyclohexylamin als Katalysator wurden vermischt.

100 g dieser Mischung wurden mit

15 g 1,1,1,4,4,4-Hexafluorbutan als Treibmittel mittels Laborrührer intensiv vermischt.

Dieses Gemisch wurde mit 152 g rohem 4,4'-Diisocyanatodiphenylmethan verschäumt. Es entstand ein harter Polyurethanschaumstoff. Verschäumungs- und physikalische Daten:

```
Liegezeit          (s)          : 10
Abbindezeit        (s)          : 44
freie Rohdichte    (kg/m³)      : 25
Zellbild                        : fein
```

### Beispiel 2

60 g eines Polyethers der Hydroxylzahl 950, welcher durch Anlagerung von Propylenoxid an Trimethylpropan erhalten worden ist,

40 g eines Polyethers der Hydroxylzahl 56, der durch Anlagerung von Propylenoxid an Trimethylpropan erhalten worden ist,

0,5 g Wasser und

2 g eines Siloxanpolyethercopolymers als Schaumstabilisator wurden vermischt.

100 g dieser Mischung wurden mit

10 g 1,1,1,4,4,4-Hexafluorbutan als Treibmittel mittels Laborrührer intensiv vermischt.

Dieses Gemisch wurde mit 164 g rohem 4,4′-Diisocyanatodiphenylmethan verschäumt. Es entstend ein harter Polyurethanschaumstoff hoher Rohdichte. Verschäumungs- und physikalische Daten:

```
Liegezeit              (s)     :  80
Abbindezeit            (s)     : 130
freie Rohdichte        (kg/m³) :  73
Gesamtrohdichte ver-   (kg/m³) : 350
dichtet
Zellbild                       : fein
```

Beispiel 3

91 g eines Polyethers der Hydroxylzahl 56, der durch Anlagerung von Propylenoxid an Trimethylolpropan erhalten worden ist,

9 g Monoethylenglykol und

0,1 g Wasser wurden vermischt.

100 g dieser Mischung wurden mit

15 g 1,1,1,4,4,4-Hexafluorbutan als Treibmittel mittels Laborrührer intensiv vermischt.

Dieses Gemisch wurde mit 56 g rohem 4,4′-Diisocyanatodiphenylmethan verschäumt. Es entstand ein zähelastischer Polyurethanschaumstoff. Verschäumungs- und physikalische Daten:

```
Liegezeit              (s)     :  35
Abbindezeit            (s)     : 105
freie Rohdichte        (kg/m³) : 127
Zellbild                       : fein
```

Beispiel 4

100 g eines Polyethers der Hydroxylzahl 56, der durch Anlagerung von Propylenoxid an Trimethylolpropan erhalten worden ist,

3 g Wasser,

1 g eines Siloxanpolyethercopolymers als Schaumstabilisator,

0,05 g Dibutylzinndilaurat wurden vermischt.

100 g dieser Mischung wurden mit

10 g 1,1,1,4,4,4-Hexafluorbutan als Treibmittel mittels Laborrührer intensiv vermischt.

Dieses Gemisch wurde mit 41 g Toluylendiisocyanat verschäumt. Es entstand ein weicher Polyurethanschaumstoff. Verschäumungs- und physikalische Daten:

```
Liegezeit              (s)     :   7
Abbindezeit            (s)     : 100
freie Rohdichte        (kg/m³) :  27
Zellbild                       : fein
```

**Patentansprüche**

1. Verwendung von 1,1,1,4,4,4-Hexafluorbutan als Treib- und Dämmgas bei der Herstellung von Kunststoff-Schaumstoffen.

2. Verwendung gemäß Anspruch 1, dadurch gekennzeichnet, daß 1,1,1,4,4,4-Hexafluorbutan in einer Menge von 2-30 Gew.-%, bezogen auf den Kunststoff-Schaumstoff, eingesetzt wird.

3. Verwendung gemäß Ansprüchen 1 und 2, dadurch gekennzeichnet, daß 1,1,1,4,4,4-Hexafluorbutan in einer Menge von 2-15 Gew.-%, bezogen auf den Kunststoff-Schaumstoff, eingesetzt wird.

4. Verwendung gemäß Ansprüchen 1 bis 3, dadurch gekennzeichnet, daß 1,1,1,4,4,4-Hexafluorbutan in einer Menge von 2-8 Gew.-%, bezogen auf den Kunststoff-Schaumstoff, eingesetzt wird.

5. Verwendung gemäß Ansprüchen 1 bis 4 bei der Herstellung von Schaumstoffen auf Isocyanatbasis.

6. Verwendung gemäß Ansprüchen 1 bis 5 bei der Herstellung von Polyurethanschaumstoffen.

7. Verwendung gemäß Ansprüchen 1-5 bei der Herstellung von Polyisocyanuratschaumstoffen.

**Claims**

1. The use of 1,1,1,4,4,4-hexafluorobutane as blowing and insulating gas in the production of foam plastics.

2. The use claimed in claim 1, characterized in that 1,1,1,4,4,4-hexafluorobutane is used in a quantity of 2 to 30% by weight, based on the foam plastic.

3. The use claimed in claims 1 and 2, characterized in that 1,1,1,4,4,4-hexafluorobutane is used in a quantity of 2 to 15% by weight, based on the foam plastic.

4. The use claimed in claims 1 to 3, characterized in that 1,1,1,4,4,4-hexafluorobutane is used in a quantity of 2 to 8% by weight, based on the foam plastic.

5. The use claimed in claims 1 to 4 in the production of isocyanate-based foams.

6. The use claimed in claims 1 to 5 in the production of polyurethane foams.

7. The use claimed in claims 1 to 5 in the production of polyisocyanurate foams.

**Revendications**

1. Utilisation de 1,1,1,4,4,4-hexafluorobutane comme agent moussant et isolant gazeux dans la fabrication de mousses de matières synthétiques.

2. Utilisation selon la revendication 1, caractérisée en ce que le 1,1,1,4,4,4-hexafluorobutane est utilisé en quantités de 2 à 30% en poids par rapport à la mousse de matière synthétique.

3. Utilisation selon les revendications 1 et 2, caractérisée en ce que le 1,1,1,4,4,4-hexafluorobutane est utilisé en quantités de 2 à 15% en poids par rapport à la mousse de matière synthétique.

4. Utilisation selon les revendications 1 à 3, caractérisée en ce que le 1,1,1,4,4,4-hexafluorobutane est utilisé en quantité de 2 à 8% en poids par rapport à la mousse de matière synthétique.

5. Utilisation selon les revendications 1 à 4 dans la fabrication de mousses à base d'isocyanates.

6. Utilisation selon les revendications 1 à 5 dans la fabrication des mousses de polyuréthannes.

7. Utilisation selon les revendications 1 à 5 dans la fabrication des mousses de polyisocyanurates.